# EUROPEAN PATENT APPLICATION

(11) **EP 2 944 315 A1**
(43) Date of publication of application: **18.11.2015**
(21) Application number: 14738010.9
(22) Date of filing: 13.01.2014
(51) Int. Cl.: A61K 35/64, A61K 33/06, A61K 31/592, A61K 31/593, A61P 19/00

(54) **METHOD AND PREPARATION FOR ACCELERATING FRACTURE HEALING**

(30) Priority: 11.01.2013 RU 2013100995
(71) Applicant: Obshestvo S Ogranichennoj Otvetstvennostju "PARAFARM", Penza 440026 (RU)
(72) Inventor: STRUKOV, VILLORIJ Ivanovich, g. Penza 440011 (RU); PROKHOROV, Mikhail Dmitrievich, g. Penza 440011 (RU); JONES-STRUKOVA, Olga, Grand Praire, Texas 75051 (US); TRIFONOV, Vjacheslav Nikolaevich, Penzenskaya obl. g. Zarechny 442960 (RU); ELISTRATOVA, Julija Anatolievna, g. Penza 440026 (RU); ELISTRATOV, Konstantin Gennadievich, g. Penza 440039 (RU); KURUS`, Natal`ja Vjacheslavovna, g. Penza 440026 (RU); EREMINA, Natalya Vyacheslavovna, g. Penza 440047 (RU); MAKSIMOVA, Marina Nikolaevna, g. Penza 440044 (RU); GALEEVA, Ramziya Timurshovna, g. Penza 440062 (RU); RADCHENKO, Larisa Grigorievna, g. Penza 440062 (RU); FEDOROV, Aleksandr Viktorovich, Penzenskaya obl. g. Kuznetsk 442530 (RU); KRUTYAKOV, Evgeny Nikolaievich, g. Penza 440062 (RU); ANDREEVA, Elena Stanislavovna, g. Penza 440062 (RU); ELISTRATOVA, Tatiana Viktorovna, g. Penza 440062 (RU); KHOMYAKOVA, Irina Vladimirovna, g. Penza 440062 (RU); TOLBINA, Galina Anatolievna, g. Penza 440444 (RU)
(74) Representative: Moffat, John Andrew
(86) International application number: PCT/RU2014/000008
(87) International publication number: WO 2014/109677

(57) **Abstract**

Invention relates to medicine, and may be used for accelerated consolidation of bone fractures. The technical result to which is aimed the claimed invention is to accelerate consolidation of bone fractures, reducing of recovery and disability terms in patients.

## Description

Invention relates to medicine, and may be used for accelerated consolidation of bone fractures.

It is known a method of accelerated consolidation of diaphyseal fractures of shank bones (Patent Nr. 2158566, Janyary 15, 1996). For this purpose from the first day of treatment patient is being injected with heparin intramuscularly 5000 units four times per day during 5-7 days. Simultaneously he is being administered with phenylin according the following dosage regimen: 0.03 g three times per day during 3 days, then the same dose two times per day during 2 days, and, thereafter, 0.015 g two times per day during 3 days. Furthermore, patient is being administered with aspirin: 0.5 g three times per day during 5 days, thereafter 0.5 g two times per day during 5 days. Then, during 7-10 days the fracture area is being electrophoresezed with heparin and euphylline. This method allows prevent delayed bone consolidation or non-consolidation as well as accelerate the process of bone consolidation.

The main disadvantage of this method is its durance and complexity of the whole process of healing.

Problem of stimulating of osteogenesis along with search of ways for bone tissue regeneration control is one of the most topical, especially in orthopedy / traumatology and veterinary science (See: Bagdonovich U.Ya., Akberdina D.L., 1976; Kaem R.I., Karlov V.D., 1981; Lavrishcheva G.I., Onoprienko G.A., 1996; Derevianko I.V., 2001). This fact is due to a great numbers of patients with orthopedic and traumatologic lesions, complicated with oppression of reparative osteogenesis, as well as with other diseases of musculoskeletal system whose pathogenesis is due to an imbalance of the normal bone formation (osteoporosis, for example).

It is known that a general biological stimulation of reparatory processes (including bone tissue) is being obtained by parenteral administration of, albumin, protein containing and anabolic medications, use of pyrimidine and purine derivatives (pentoxyl, methyluracil, calcium orotate) (See: Rusakov V.I., 1976, Bulovetsky et al., 1981). What concerns bone tissue, in addition to general biological stimulating of reparatory processes there is used medicaments which make up deficiency on calcium in organism and thereby stimulate the phase of calcification of bone matrix without affecting the rate of formation of protein matrix of a bone. The rate of osteogenesis in whole depends just on the rate of formation of this protein matrix (See: Slutsky, 1969).

The nearest technical solution for claimed invention is a medicament "Osteogenon" that is used for treatment of various forms of osteoporosis as well as for accelerated healing of bone fractures (See: RLS - Encyclopedia of medicaments, 2001, page 656).

"Osteogenon" is administered in the case of systemic osteoporosis orally 2-4 tablets (one coated tablet, 830 mg), but for accelerating of fracture healing may be used 1-2 tablets per day (See: RLS - Encyclopedia of medicaments, 2001, page 656). According to the opinion of the creators of "Osteogenon" which is being produced from bone tissue of animals this medicament stops or reduces resorption of bone tissue (hamper osteoclasts) and stimulates bone formation (activates osteoblasts) (See: RLS - Encyclopedia of medicaments, 2001, page 1110). However, there may noted its disadvantages: this medicament is effective only with prolonged use (from some months to one year), it may provoke exacerbation of the disease in patients with urolithiasis, it needs control of the levels of calcium and phosphor in urine, and it is too expensive.

Technical result to which is aimed claimed invention is to accelerate consolidation of bone fractures, reducing of recovery and disability terms in patients.

This technical result is being obtained by administering of drone brood and calcium compound. According to the invention, drone brood and calcium compound must be taken in simultaneously during 24 hours. Calcium compound and drone brood for treatment of arthritis and osteoarthrosis may be in form of form of powder, tablets or capsules, wherein calcium compound is one from the following group of compounds or any combination of them - calcium carbonate, calcium citrate, calcium gluconate, calcium aspartate, calcium ascorbate, calcium aminochelate, calcium fumarate, calcium succinate, and calcium phosphate and calcium citrate.

The average losses in the sphere of socially useful work caused by bone fractures in the scale of the whole country, even in very rough estimates, are huge - many millions of working days. And if specialists could at least on one third accelerate processes of bone consolidation, it would be greatly useful for people and society. According to statistic data, only in Penza city, Russia, in hospitals are treated in average about 50.000 patients with various injuries. Of these, about 60% are injuries which do not demand hospitalization (fractures without displacement or with slight displacement), about 40% are fractures which demand hospitalization, including operative treatment (about 10%).

For the last time there is a tendency to increase of terms of immobilization of fractures due to the slow bone consolidation that, in turn, increases terms of recovery and disability in patients.

It is known that consolidation of bone fractures is accompanied by series of local and general biological changes. So, may be noted 5 phases of regeneration of bone tissue.

**First phase:** formation of embryonic (mesenchimal) tissue whose growth begins directly after injury. In the area of bone fracture forms a kind of "primary adhesive" from hematoma (edematous fluid and fibrin).

**Second phase:** differentiation of cell elements of hematoma and formation of fibrous structures accompanied with formation of cell-fibrous tissues on the base of which further will be accumulated the bone substance.

**Third phase:** deposition of bone tissue. In collagenous fibers of connective tissue callus begins formation of areas of sealing with formation of solid mass because of deposition of proteins which are the base of primitive osteoid beamses - initially isolated, and then in form of a dense network.

**Fourth phase:** formation and calcification of bone callus. Ossification of callus is effectuating predominantly due to blood calcium where it comes from the whole skeletal system, including neighboring with fracture bone areas.

**Fifth phase:** reconstruction of callus accompanied with substitution of immature bone structures by more mature ones and adaptation to pressure on the bone. The bone callus is being reconstructed according to functional requirements to it. Some structures are resorbing and other are forming and strengthening. Reconstruction of final callus lasts months and even years that depends on the position of knitted bone fragments, dimension of callus, and conformity of the axis of the injured limb with functional requirements of pressure on the bone.

Various sources mention that recovery after bone fracture may be disturbed at any phase of bone callus formation, for example, in the case of great hematoma, bad condition of bone fragments, osteoporosis, deficiency on calcium in organism, that is often associated with unhealthy lifestyle (smoking, alcohol, sedentary lifestyle, little exposure to sunlight), or unhealthy diet (insufficient consumption of products containing calcium and phosphor). All these factors influence on processes of bone consolidation and on bone density. In connection with the foregoing, scientific works containing studies of mechanisms of normalization or acceleration of processes of bone consolidation, as well as development of new medicaments on their base have great scientific and practical importance.

Research of drone brood properties established its ability to accelerate consolidation of bone fractures due to the enhancing of functioning of osteoblasts which are responsible for bone tissue formation.

This assumption was tested in practice.

### Materials and methods

For investigation were selected 300 patients (men - 112, women - 188) in the age from 18 to 62 years with the rarest types of fractures, such as fracture of distal metaepiphysis of radial bone or fracture of ankle lateral malleoulus.

All patients were divided in three groups:
1st group (122 patients) was treated with calcium compound and drone brood (conventionally we defined this composition as "Treatment For Fractures", TFT), dosage - 3 tablets two times per day;
2nd group (103 patients) was treated with "Calcium D3 Nycomed", dosage - 1 tablet two times per day;
3rd group (75 patients) received no calcium supplements.

All patients underwent an X-ray examination before applying of gypsum plaster and after its removal. All patients received products with high content of calcium. For the study were selected average terms of immobilization, i.e. 4 weeks.

### Results:

- The patients which received no calcium supplements showed no signs of formation of bone callus (formation of bone callus became evident only on the 5th week);
- The patients treated with "Calcium D3 Nycomed" demonstrated little bone consolidation without formation of bone callus (formation of bone callus became evident only on 4th - 5th week after immobilization);
- The patients treated with TFT demonstrated formation of bone callus (on roentgenogram).

These results suggest that patients treated with TFT were able to begin rehabilitation 3 weeks later after the injury, and one month later to return to their work.

The patients treated with "Calcium D3 Nycomed" were able to begin rehabilitation only 4 weeks later after the injury, and 1.5 month later to return to their work.

The patients which received no calcium supplements begun their rehabilitation only 1.5 month after injury, and returned to their work after 2 months after injury.

Based on the above mentioned, it can be concluded that the use of TFT composition for treatment of patients with fractures reduces the terms of immobilization. It allows early rehabilitation with fractures, i.e. early rehabilitation with return to workplace. Undoubtedly, it has great economic importance.

And, however, the use of drone brood and calcium supplements is known from prior art, this composition intended for accelerating of consolidation of fractures was not known and used earlier.

## Claims

1. Method of accelerated consolidation of bone fractures which includes intake of drone brood and calcium compounds.

2. Method from the Claim 1 which is **characterized in that** drone brood and calcium compounds are taken in simultaneously during 24 hours.

3. Composition for accelerated consolidation of bone fractures which comprises drone brood and calcium compounds.

4. Composition from the Claim 3 which is **characterized in that** it is made a form of powder, tablets or capsules.

5. Composition from the Claim 3 which is **characterized in that** a calcium compound is one from the following group of compounds or any combination of them - calcium carbonate, calcium citrate, calcium gluconate, calcium aspartate, calcium ascorbate, calcium aminochelate, calcium fumarate, calcium succinate, and calcium phosphate and calcium citrate.
